# EUROPEAN PATENT APPLICATION

(11) **EP 2 020 246 A1**
(43) Date of publication of application: **04.02.2009**
(21) Application number: 07075665.5
(22) Date of filing: 03.08.2007
(51) Int. Cl.: A61M 1/10

(54) **Control of rotary blood pump with selectable therapeutic options**

(71) Applicant: Berlin Heart GmbH, 12247 Berlin (DE)
(72) Inventor: Arndt, Andreas, 12555 Berlin (DE); Graichen, Kurt, 13189 Berlin (DE); Nüsser, Peter, 13051 Berlin (DE)
(74) Representative: Pfenning, Meinig & Partner GbR

(57) **Abstract**

The present invention refers to a method to control a rotational blood pump, characterized by direct or indirect measurement of the pressure difference across the pump, calculating an index of pulsatility (PI) of the pressure difference, estimating the gradient of PI with respect to rotational speed (n) dPI/dn and relulating the dPI/dn to a pre-defined set-point.

## Description

A control strategy for rotary blood pumps is proposed which satisfies different user-selectable control objectives: maximum degree support with the highest possible flow rate versus medium degree of support but maximum ventricular wash-out and controlled opening of the aortic valve.

### Disclosure of the invention

An index of pulsatility (PI) is calculated from the pressure difference signal measured by the pump. The gradient PI / pump speed (dPI/dn) is estimated via on-line parameter identification. The gradient dPI/dn is extracted from the system dynamics which is estimated by an on-line parameter identification method. The outer loop of a cascaded adaptive controller regulates the dPI/dn to a reference value that satisfies the selected control objective. The set-point of dPI/dn is selected in such a way that the pump operates in the transition region between an opening and a closed aortic valve, this region being at the transition point between partial and total assist. The rotational speed may be reduced temporarily from the operating point by a fixed value to allow the aortic valve (or pulmonary valve respectively) to open in systole. The outer loop comprises a feedback control loop that keeps the dPI/dn to its set-point and whose output is a reference value for the PI. The gradient of PI may be estimated with respect to rotational speed (n) dPI/dn and the pump may be regulated in a way that the dPI/dn is minimal. The minimum of dPI/dn is then maintained by the cascaded controller and the feedback control loop keeps the dPI/dn to its minimum value and its output is a reference value for the PI.

The inner loop controls the PI to a reference value set by the outer loop. The inner feedback control loop keeps the actual PI close to the reference value for PI by calculating a reference value for the rotational speed. Adverse pumping states like suction and regurgitation can be identified through the estimate of dPI/dn and reverted by the controller.

A lumped-parameter computer model of the assisted circulation has been used to simulate variations of ventricular contractility, pulmonary venous pressure, aortic pressure and heart rate.

A safe and optimal reference value for PI was calculated every 30 s. The actual PI was controlled continuously at the set-point by the inner loop. For maximum support a low PI was maintained without inducing ventricular collapse. For maximum wash-out the pump worked in the transition region between an opening and a closed aortic valve at a high PI. From this operating point excursions to a lower speed are possible, where the aortic valve predictably opens.

## Claims

1. A method to control a rotational blood pump, **characterized by** direct or indirect measurement of the pressure difference across the pump, calculating an index of pulsatility (PI) of the pressure difference, estimating the gradient of PI with respect to rotational speed (n) dPI/dn and regulating the dPI/dn to a pre-defined set-point.

2. A method according to claim 1, wherein the gradient dPI/dn is extracted from the system dynamics which is estimated by an on-line parameter identification method.

3. A method according to claim 1, wherein the set-point of dPI/dn is selected in such a way that the pump operates in the transition region between an opening and a closed aortic valve, this region being at the transition point between partial and total assist.

4. A method according to claim 1, wherein the dPI/dn is regulated to the set-point by a cascaded controller.

5. A method according to claim 4, wherein the outer loop comprises a feedback control loop that keeps the dPI/dn to its set-point and whose output is a reference value for the PI.

6. A method according to claims 4 and 5, wherein an inner feedback control loop keeps the actual PI close to the reference value for PI by calculating a reference value for the rotational speed.

7. A method according to claims 1 and 3, wherein the rotational speed is reduced temporarily from the operating point by a fixed value to allow the aortic valve (or pulmonary valve respectively) to open in systole.

8. A method to control a rotational blood pump, **characterized by** direct or indirect measurement of the pressure difference across the pump, calculating an index of pulsatility (PI) of the pressure difference, estimating the gradient of PI with respect to rotational speed (n) dPI/dn and regulating the pump in a way that the dPI/dn is minimal.

9. A method according to claim 8, wherein the gradient dPI/dn is extracted from the system dynamics that is estimated by an on-line parameter identification method.

10. A method according to claim 8, wherein the minimum of dPI/dn is maintained by a cascaded controller.

11. A method according to claim 8 and 10, wherein the outer loop comprises a feedback control loop that keeps the dPI/dn to its minimum value and whose output is a reference value for the PI.

12. A method according to claims 10 and 11, wherein an inner feedback control loop keeps the actual PI close to the reference value for PI by calculating a reference value for the rotational speed.

13. A method according to claims 1 through 12, wherein the parameters of the inner feedback control loop are adapted to the estimated system dynamics.
